# EUROPEAN PATENT APPLICATION

(11) **EP 4 599 753 A1**
(43) Date of publication of application: **13.08.2025**
(21) Application number: 23889210.3
(22) Date of filing: 10.11.2023
(51) Int. Cl.: A61B 5/00, G16H 50/20, G06N 20/20

(54) **ELECTRONIC DEVICE FOR DIAGNOSING DISEASE OF USER ON BASIS OF BIOLOGICAL SIGNAL, AND CONTROL METHOD THEREFOR**

(30) Priority: 11.11.2022 KR 20220150322; 09.11.2023 KR 20230154684
(71) Applicant: Medical AI Co., Ltd., Seoul 06180 (KR)
(72) Inventor: KWON, Joonmyoung, Seoul 06180 (KR); JO, Yongyeon, Seoul 06180 (KR)
(74) Representative: Patentanwaltskanzlei Matschnig & Forsthuber OG
(86) International application number: PCT/KR2023/018074
(87) International publication number: WO 2024/101954

(57) **Abstract**

The present disclosure provides an electronic device and a method for controlling the same. An electronic device according to one embodiment of the present disclosure includes: a communication interface; memory configured to store pre-trained first and second neural network models; and at least one processor configured to obtain biometric-data of a user, generate first information about a first disease of the user by inputting the obtained biometric-data to a pre-trained first neural network model, generate second information about the first disease of the user by inputting the obtained biometric-data to a pre-trained second neural network model, and generate a diagnosis result for the user regarding a first category of the first disease based on the first and second information.

## Description

### Technical Field

The present disclosure relates to an electronic device for diagnosing a disease of a user based on a biosignal, and a method for controlling the same. More specifically, the present disclosure relates to an electronic device for diagnosing a disease of a user using a pre-trained neural network model, and a method for controlling the same.

### Background Art

Myocardial infarction and ischemic heart disease are the most common causes of sudden death, and the number of patients with myocardial infarction and ischemic heart disease has been increasing every year recently. Since early detection and treatment of myocardial infarction and ischemic heart disease can prevent death and disability, there are being conducted many studies on early diagnosis and prediction of myocardial infarction and ischemic heart disease.

For early diagnosis of myocardial infarction and ischemic heart disease, there is widely used a method of measuring an electrocardiogram, displaying a measured electrocardiogram signal in the form of a graph, and determining whether the presence or absence of myocardial infarction and ischemic heart disease in a patient's heart based on the graph. In particular, recently, with the development of deep learning technology, there has been increasing interest in a method of obtaining an electrocardiogram signal analysis result only by inputting the graph of the electrocardiogram signal to a neural network model through the application of deep learning technology to the medical field.

### Disclosure

### Technical Problem

The present disclosure has been conceived in response to the above-described background art, and an object of the present disclosure is to provide an electronic device for diagnosing a disease of a user based on a pre-trained neural network model and a biosignal of the user, and a method for controlling the same.

However, the objects to be achieved in the present disclosure are not limited to the object mentioned above, and other objects not mentioned may be clearly understood based on the following description.

### Technical Solution

According to one embodiment of the present disclosure for achieving the above-described object, there is disclosed a method that is performed by an electronic device. The method includes: obtaining biometric-data of a user; generating first information about a first disease of the user by inputting the obtained biometric-data to a pre-trained first neural network model; generating second information about the first disease of the user by inputting the obtained biometric-data to a pre-trained second neural network model; and generating a diagnosis result for the user regarding a first category of the first disease based on the first and second information.

Alternatively, generating the diagnosis result may include: adjusting a first probability value included in the first information based on a second probability value included in the second information, and generating a diagnosis result for the user regarding the first disease based on the first probability value.

Alternatively, generating the diagnosis result may include: when the second probability value is equal to or larger than a reference value, adjusting the first probability value by applying a weight corresponding to the second probability value to the first probability value; and, when the second probability value is smaller than the reference value, maintaining the first probability value.

Alternatively, generating the diagnosis result may include: generating a first diagnosis result corresponding to the first disease when the first probability value is equal to or larger than a first value, generating a second diagnosis result corresponding to the first disease when the first probability value is smaller than the first value and equal to or larger than a second value, and generating a third diagnosis result corresponding to the first disease when the first probability value is smaller than the second value.

Alternatively, the first information may be information about the presence or absence of the first disease, and the second information may be information about a first type of the first disease.

Alternatively, the method may include extracting first and second neural network models from among a plurality of neural network models based on the first disease and the category of the diagnosis result.

Alternatively, generating the information regarding the presence or absence of the first disease and generating the information regarding the first type may be performed in parallel.

Alternatively, the first and second neural network models may be pre-trained based on training data in which a plurality of labels set based on different criteria are assigned for a same electrocardiogram signal, and the plurality of labels may include a first-category label corresponding to the presence or absence of the first disease and a second-category label corresponding to the first type of the first disease.

Alternatively, the biometric-data may include an electrocardiogram signal, the first disease may include any one of myocardial infarction and ischemic heart disease, and the first type may include any one of ST elevation myocardial infarction (STEMI) and non-ST elevation myocardial infarction (NSTEMI).

According to one embodiment of the present disclosure for achieving the above-described object, there is disclosed an electronic device. The electronic device includes: a communication interface; memory configured to store pre-trained first and second neural network models; and at least one processor configured to obtain biometric-data of a user, generate first information about a first disease of the user by inputting the obtained biometric-data to a pre-trained first neural network model, generate second information about the first disease of the user by inputting the obtained biometric-data to a pre-trained second neural network model, and generate a diagnosis result for the user regarding a first category of the first disease based on the first and second information.

According to one embodiment of the present disclosure, there is disclosed a non-transitory computer-readable storage medium storing computer instructions that, when executed by a processor of an electronic device, cause the electronic device to perform operations, wherein the operations include: obtaining biometric-data of a user; generating first information about a first disease of the user by inputting the obtained biometric-data to a pre-trained first neural network model; generating second information about the first disease of the user by inputting the obtained biometric-data to a pre-trained second neural network model; and generating a diagnosis result for the user regarding a first category of the first disease based on the first and second information.

### Advantageous Effects

In the present disclosure, the electronic device according to one embodiment of the present invention may train a plurality of neural network models to generate information about the presence or absence of a disease and information about the type of disease by using the same training data, thereby reducing the cost and time required to secure and preprocess training data.

In addition, information about a disease of a user and the type of disease is provided only by measuring a biosignal of the user, leading to the prediction and early diagnosis of a disease of the user.

### Description of Drawings

FIG. 1 is an exemplary diagram of an electronic device according to one embodiment of the present disclosure;
FIG. 2 is a block diagram of an electronic device according to one embodiment of the present disclosure;
FIG. 3 is a flowchart schematically showing a method for controlling an electronic device according to one embodiment of the present disclosure;
FIG. 4 is an exemplary diagram showing pre-trained first and second neural network models stored in an electronic device according to one embodiment of the present disclosure;
FIG. 5a is an exemplary diagram showing a method for training first and second neural network models (20-1 and 20-2) according to one embodiment of the present disclosure, and FIG. 5b is an exemplary diagram showing a method for training multiple neural network models in order to generate information about the presence or absence of a specific disease and information about a specific type of a specific disease;
FIG. 6 is a flowchart showing a method for diagnosing a disease of a user by using first and second neural network models according to one embodiment of the present disclosure; and
FIG. 7 is a detailed configuration diagram of an electronic device according to an alternative embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein may be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein may be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, they are not used in the sense of limiting the technical spirit of the present disclosure.

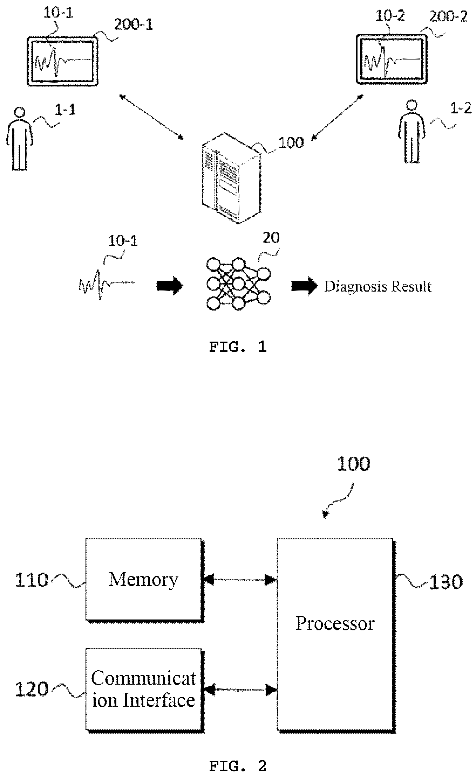

FIG. 1 is an exemplary diagram of an electronic device according to one embodiment of the present disclosure.

Referring to FIG. 1, an electronic device 100 according to one embodiment of the present disclosure obtains biosignals 10 of users from external electronic devices 200 that operate in conjunction with the electronic device 100. In this case, the external electronic devices 200-1 and 200-2 (hereinafter, 200) that operate in conjunction with the electronic device 100 are connected to the electronic device 100 through network communication, and may be implemented as various devices that each perform a function of measuring a biosignal 10-1 or 10-2 (hereinafter, 10) of a user 1-1 or 1-2 (hereinafter, 1). For example, the external electronic device 200 may be implemented as an electrocardiogram measurement device, a smart watch, a display device, or the like. Meanwhile, the external electronic device 200 may register information about the external electronic device 200 (or information about an institution in which the external electronic device 200 is disposed) in the electronic device 100 in advance in order to operate in conjunction with the electronic device 100.

The electronic device 100 may generate a diagnosis result for a user based on a user signal obtained from the external electronic device 200. More specifically, the electronic device 100 may determine the health status of the user or identify the presence or absence of a disease, type of disease, and like of the user by analyzing the user signal obtained from the external electronic device 200. In particular, in order to generate a diagnosis result for the user, the electronic device 100 may use a pre-trained neural network model 20. As an example, the electronic device 100 may obtain the user's health status information or the user's disease information (e.g., information including the presence or absence of a disease, the type of disease, or the like) as an output value of the pre-trained neural network model 20 by inputting the obtained biosignal 10 to the pre-trained neural network model 20. To this end, the pre-trained neural network model 20 may be pre-trained to output the user's health status information, the user's disease information, and/or the like based on various user biosignals 10.

Meanwhile, the electronic device 100 may transmit the generated diagnosis result to the external electronic device 200, or may transmit it to a user terminal device. Through this, the user may identify health status information only by measuring the biosignal 10 without the help of a specialized facility or expert.

Referring to FIGS. 2 to 7, an electronic device 100 according to one embodiment of the present disclosure will be described in detail below.

FIG. 2 is a block diagram of an electronic device 100 according to one embodiment of the present disclosure.

The electronic device 100 according to the one embodiment of the present disclosure may be a hardware device or a part of a hardware device that performs the comprehensive processing and computation of data, or may be a software-based computing environment connected over a communication network. For example, the electronic device 100 may be a server (e.g., a platform server, or the like) that performs an intensive data processing function and shares resources, or may be a client that shares resources through interaction with a server. Furthermore, the electronic device 100 may be a cloud system in which pluralities of servers and clients comprehensively process data while interacting with each other.

The above description is only one example related to the type of electronic device 100, so that the type of electronic device 100 can be constructed in various manners within a range understandable to those skilled in the art based on the content of the present disclosure. For example, the electronic device 100 may be implemented as an electronic device 100 that measures a user's biosignal (e.g., an electrocardiogram signal, or the like). However, the following description will be given on the assumption that the electronic device 100 of the present disclosure is a server.

Referring to FIG. 2, the electronic device 100 according to the one embodiment of the present disclosure may include memory 110, a communication interface (a network unit) 120, and at least one processor 130. However, FIG. 2 shows only an example, and the electronic device 100 may include other components for implementing a computing environment. Furthermore, only some of the disclosed components may be included in the electronic device 100.

The memory 110 according to one embodiment of the present disclosure may be understood as a constituent unit including hardware and/or software for storing and managing the data processed in the electronic device 100. That is, the memory 110 may store any form of data generated or determined by the processor 130 and any form of data received by the processor 130 via the communication interface 120.

As an example, the memory 110 may store a plurality of pre-trained neural network models. Each of the neural network models may be matched with at least one other neural network model according to the category of a disease to be diagnosed and the category of a diagnostic result to be generated, and the matching information may be stored in the memory 110. In this case, the plurality of matched neural network models may be trained to output information of different categories based on the same input (e.g., a biosignal, or like). For example, the memory 110 may store first and second neural network models. In this case, the first and second neural network models may be pre-trained based on the same training data and then stored in the memory 110, and the pre-trained first and second neural network models may be trained to generate information of different categories from a biosignal of the user by analyzing the biosignal. In this case, the memory 110 may also store training data used to train the first and second neural network models.

Furthermore, as an example, the memory 110 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 110 may include a database system that controls and manages data in a predetermined system. Since the types of memory 110 described above are only examples, the type of memory 110 may be configured in various manners within a range understandable to those skilled in the art based on the content of the present disclosure.

The communication interface 120 according to one embodiment of the present disclosure may be understood as a constituent unit that transmits and receives data via any known wired/wireless communication system. The electronic device 100 may transmit and receive various types of information to and from the external electronic device 100 via the communication interface 120. As an example, the electronic device 100 may receive the user's biosignal, measured by the external electronic device 100, from the external electronic device 100 via the communication interface 120, or may transmit the user's diagnosis result information, generated by the electronic device 100, to the external electronic device 100.

To this end, the communication interface 120 may perform data transmission and reception by using a wired/wireless communication system such as a local area network (LAN), a wideband code division multiple access (WCDMA) network, a long term evolution (LTE) network, the wireless broadband Internet (WiBro), a 5th generation mobile communication (5G) network, an ultra-wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the communication interface 120 may be applied in various manners other than the above-described examples.

The processor 130 according to one embodiment of the present disclosure is electrically connected to the memory 110 and the communication interface 120, and may control the overall operation of the electronic device 100.

The processor 130 according to one embodiment of the present disclosure may be understood as a constituent unit including hardware and/or software for performing computing operations. For example, the processor 130 may read a computer program and perform data processing for machine learning. The processor 130 may process operation processes such as the processing of input data for machine learning, the extraction of features for machine learning, and the computation of errors based on backpropagation. The processor 130 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 130 described above are only examples, the type of processor 130 may be configured in various manners within a range understandable to those skilled in the art based on the content of the present disclosure.

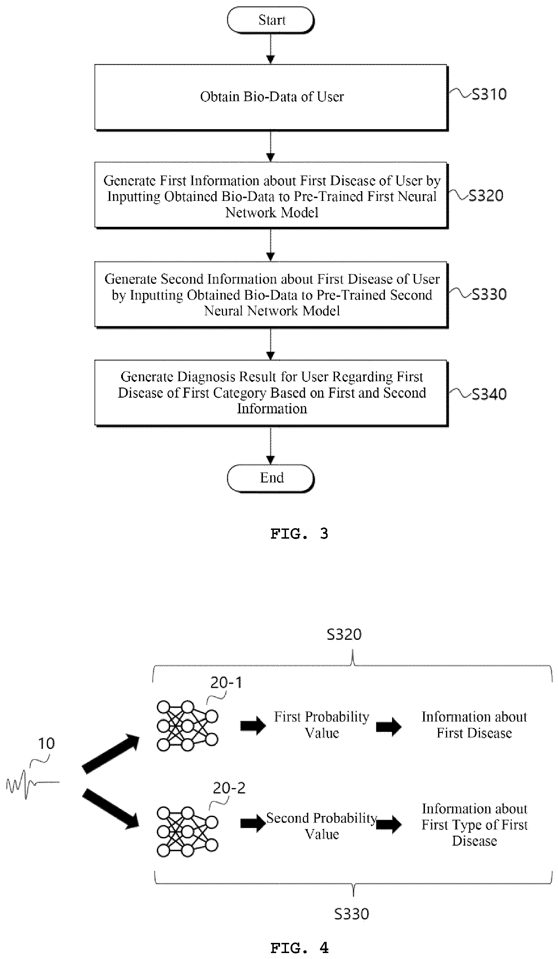

FIG. 3 is a flowchart schematically showing a method for controlling the electronic device 100 according to one embodiment of the present disclosure.

Referring to FIG. 3, the processor 130 obtains biometric-data of the user 1 in step S310. More specifically, the processor 130 obtains biometric-data of the user 1 from the external electronic device 200, registered in the electronic device 100, via the communication interface 120. In this case, information about the external electronic device 200 may be stored in the memory 110 of the electronic device 100.

As an example, the biometric-data of the user 1 may be an electrocardiogram signal. More specifically, the external electronic device 200 may be an electrocardiogram device including a plurality of electrodes (or a plurality of patches including respective electrodes) configured to be attached to different parts of the body of the user 1. The external electronic device 200 may generate an electrocardiogram signal (or electrocardiogram data) corresponding to the user 1 based on the electrocardiogram of the user 1 measured from individual electrodes, and may transmit it to the electronic device 100. Furthermore, the processor 130 may obtain the electrocardiogram signal, generated by the external electronic device 200, via the communication interface 120. For the purpose of understanding the present disclosure, the following description will be given on the assumption that the biometric-data is an electrocardiogram signal.

Referring to FIG. 3, the processor 130 may generate first information about a disease of the user 1 by inputting the obtained biometric-data to the pre-trained first neural network model in step S320.

For example, the first information may be information about the presence or absence of a disease. Accordingly, the first neural network model may be a model that has been pre-trained to generate information about the presence or absence of a disease of the user 1. More specifically, the first neural network model may be a model that has been pre-trained to, when an electrocardiogram signal of the user 1 is input, determine whether a disease is present in the user 1 based on the electrocardiogram signal and generate information about the presence or absence of the disease in the user 1.

In particular, the first neural network model may be pre-trained to determine only the presence or absence of a specific disease. Hereinafter, for ease of description of the present disclosure, the specific disease will be referred to as a first disease.

As an example, when an electrocardiogram signal of the user 1 is input, the first neural network model may determine whether the user 1 has a first disease based on the electrocardiogram signal, and may generate information about the presence or absence of the first disease as an output value.

In this case, the information about whether the first disease is present may include the probability value (or the score) of the presence of the first disease. More specifically, when an electrocardiogram signal of the user 1 is input to the first neural network model, the processor 130 may obtain the probability value of the presence of the first disease as an output value via a Sigmoid Layer or SoftMax Layer disposed in an output stage out of a plurality of layers included in the first neural network model.

In addition, the processor 130 may generate second information about the disease of the user 1 by inputting the obtained biometric-data to the pre-trained second neural network model in step S330.

As an example, the second information may be information about the type of disease. Accordingly, the second neural network model may be a model that has been pre-trained to generate information about the type of disease of the user 1. More specifically, the second neural network model may be a model that has been pre-trained to, when an electrocardiogram signal of the user 1 is input, identify the type of disease of the user 1 based on the electrocardiogram signal and generate information about the type of disease of the user 1.

In particular, the second neural network model may be pre-trained to generate information about the type of specific disease. In this case, the specific disease may be the same disease (i.e., the first disease) as the disease for which the first neural network model identifies the presence or absence thereof.

In addition, the second neural network model may be pre-trained to generate information about a specific type of a specific disease. That is, when there are various types related to a specific disease, the second neural network model may be pre-trained to identify whether the specific disease of the user 1 corresponds to a specific one of the various types related to the specific disease. In particular, the second neural network model may be trained to identify the characteristics of a biosignal by analyzing the biosignal and then identify a specific type of a specific disease corresponding to the identified characteristics. For ease of description of the present disclosure, the specific type will be referred to as the first type below.

As an example, when an electrocardiogram signal of the user 1 is input, the second neural network model may determine whether the first disease of the user 1 corresponds to the first type based on the electrocardiogram signal, and may generate information about the first type of the first disease as an output value.

In this case, the information about the first type of the first disease may include the probability value (or the score) at which the disease of the user 1 corresponds to the first type of the first disease. More specifically, when an electrocardiogram signal of the user 1 is input to the second neural network model, the processor 130 may obtain, as an output value, a probability value at which the disease of the user 1 corresponds to the first type of the first disease via a Sigmoid Layer or SoftMax Layer disposed in an output stage out of a plurality of layers included in the second neural network model.

As an example, the first and second neural network models each may be implemented as a convolution neural network (CNN) model, a recurrent neural network (RNN) model, or a Relu model.

Meanwhile, the processor 130 may perform a preprocessing process for an obtained biosignal 10 (e.g., an electrocardiogram signal) before inputting the obtained biosignal 10 to the first and second neural network models.

Meanwhile, according to one embodiment of the present disclosure, a plurality of neural network models may be stored in the memory 110. In this case, the plurality of neural network models may be matched with other neural network models according to the category of a disease and the category of a diagnosis result. Accordingly, the processor 130 may identify the category of a disease to be diagnosed and the category of a diagnosis result (or the category of a disease and category of a diagnosis result set by the user), and may determine a combination of multiple neural network models, corresponding to the identified category of the disease and category of the diagnosis result, out of the plurality of neural network models. Then, the processor 130 may extract the multiple neural network models according to the determined combination and then generate a diagnosis result regarding a specific disease for the user 1 by using the extracted multiple neural network models. Meanwhile, information about the combination of the multiple neural network models corresponding to the category of the disease and the category of the diagnosis result may be stored in the memory 110 in the form of a table.

For example, when it is determined that a diagnosis result for a first category regarding the first disease is generated, the processor 130 may extract a first neural network model and a second neural network model from among the plurality of neural network models stored in the memory 110. Then, the processor 130 may generate the diagnosis result for the first category regarding the first disease for the user 1 by inputting the obtained biosignal to each of the extracted first and second neural network models.

FIG. 4 is an exemplary diagram showing the trained first and second neural network models 20-1 and 20-2 stored in the electronic device 100 according to one embodiment of the present disclosure.

Referring to FIG. 4, the processor 130 may input an obtained electrocardiogram signal to each of the first and second neural network models 20-1 and 20-2. In this case, the processor 130 may obtain multiple probability values via the respective neural network models (the first and second neural network models 20-1 and 20-2). More specifically, the processor 130 may obtain a probability value (hereinafter, the first probability value) at which the user 1 has a first disease via the first neural network model 20-1, and may obtain a probability value (hereinafter, the second probability value) at which the user 1 has a disease corresponding to the first type of the first disease via the second neural network model 20-2.

In particular, referring to FIG. 4, the processor 130 may perform the step of generating information about the presence or absence of the first disease and the step of generating information about the first type of the first disease in parallel. That is, the processor 130 obtains the output values (i.e., the first and second probability values) of the first and second neural network models 20-1 and 20-2 by inputting an obtained electrocardiogram signal to each of the first and second neural network models 20-1 and 20-2 arranged in parallel, thereby reducing the time required to generate information about the presence or absence of the first disease and information about the first type of the first disease.

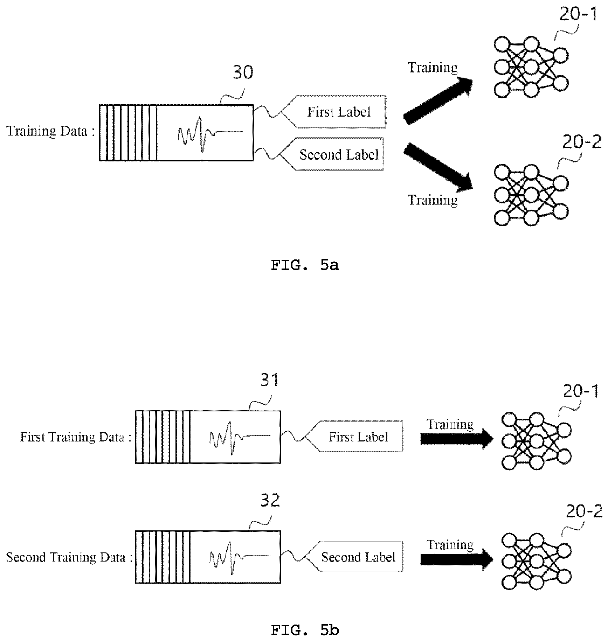

FIG. 5a is an exemplary diagram showing a method for training the first and second neural network models 20-1 and 20-2 according to one embodiment of the present disclosure, and FIG. 5b is an exemplary diagram showing a method for training multiple neural network models in order to generate information about the presence or absence of a specific disease and information about a specific type of a specific disease.

According to one embodiment of the present disclosure, the first and second neural network models 20-1 and 20-2 may be pre-trained based on training data in which multiple labels set based on different criteria are assigned for the same electrocardiogram signal.

More specifically, the first and second neural network models 20-1 and 20-2 may be pre-trained using the same training data. As an example, the training data may include a plurality of electrocardiogram signals, and may be referred to as a training data group. The first and second neural network models 20-1 and 20-2 may be trained based on the same training data including a plurality of electrocardiogram signals. However, the plurality of electrocardiogram signals included in the training data may each be given different labels in order to generate different types of information (information about the presence or absence of the first disease and information about the first type of the first disease).

In this case, the different labels are labels set based on different criteria, and may include a first-category label corresponding to the presence or absence of the first disease and a second-category label corresponding to the first type of the first disease. That is, referring to FIG. 5a, the training data according to one embodiment of the present disclosure may each be given a first-category label corresponding to the presence or absence of the first disease and a second-category label corresponding to the first type of the first disease.

Accordingly, the first neural network model 20-1 may be trained based on the first-category label of the training data to generate information about the presence or absence of the first disease, and the second neural network model 20-2 may be trained based on the second-category label of the training data to generate information about the first type of the first disease.

Referring to FIG. 5b, in order to generate information about the presence or absence of a specific disease and information about a specific type of a specific disease, separate types of training data had to be prepared for training the respective neural network models. As a result, a problem arose in that a great deal of cost and time was required to preprocess the respective types of training data. In particular, in the case of the training data that can be obtained by a specialized device, such as biosignals 10 (e.g., electrocardiogram signals), it was difficult to prepare sufficient amounts of training data for training the respective neural network models.

However, according to one embodiment of the present disclosure, in order to generate different types of information (information about the presence or absence of the first disease and information about the first type of the first disease), the same training data assigned with a plurality of labels set based on different criteria may be utilized to train a plurality of neural network models (i.e., the first and second neural network models 20-1 and 20-2), thereby allowing the training data to be used in duplicate. Accordingly, the cost and time required for preprocessing training data may be reduced, and a sufficient amount of training data may be secured for training individual neural network models.

However, the present disclosure is not limited to the above-described embodiment. That is, the first and second neural network models **20-1** and 20-2 may be trained by different types of training data. More specifically, the first neural network model 20-1 may be trained to identify the presence or absence of myocardial infarction based on first training data including electrocardiogram data to each of which the first label (the label used to identify myocardial infarction) is assigned. The second neural network model 20-2 may be trained to identify the presence or absence of ST elevation myocardial infarction based on second training data including electrocardiogram data to each of which the second label (the label used to identify ST elevation myocardial infarction) is assigned.

Referring back to FIG. 3, the processor 130 may generate a diagnosis result for the user 1 regarding the first disease of a first category based on information about the presence or absence of the first disease and information about the first type of the first disease in step S340.

More specifically, the processor 130 may generate a diagnosis result for the first disease of the user 1 by combining information about the presence or absence of the first disease and information about the first type. As an example, the processor 130 may generate a diagnosis result for the first disease of the first category by combining information about the presence or absence of the first disease and information about the first type of the first disease. That is, when the processor 130 uses the first neural network model 20-1, it may determine only the presence or absence of the first disease. However, according to one embodiment of the present disclosure, the processor 130 may generate a diagnosis result for the first disease of the first category by combining the result of the first neural network model 20-1 (i.e., the presence or absence of the first disease) and the result of the second neural network model 20-2 (i.e., whether the corresponding disease corresponds to the first type of the first disease).

Meanwhile, the processor 130 may provide the generated diagnosis result for the first disease to the user 1 by transmitting it to the external electronic device 200 via the communication interface 120.

Meanwhile, according to one embodiment of the present disclosure, the first disease includes one of myocardial infarction and ischemic heart disease, and the first type of the first disease may be a high-risk group for myocardial infarction. That is, the processor 130 may generate information about whether the user 1 has myocardial infarction or ischemic heart disease based on the electrocardiogram signal via the first neural network model 20-1, and may generate information about the high-risk group for myocardial infarction of the user 1 via the second neural network model 20-2. For ease of description of the present disclosure, the following description will be given on the assumption that the first disease is myocardial infarction.

As an example, when the processor 130 identifies either ST elevation myocardial infarction (STEMI) or non-ST elevation myocardial infarction (NSTEMI) based on the electrocardiogram signal of the user 1, the processor 130 may determine that myocardial infarction of the user 1 falls into the high-risk group.

Accordingly, the second neural network model 20-2 may be trained to output whether the user 1 has ST elevation myocardial infarction or non-ST elevation myocardial infarction based on an input electrocardiogram signal. In this case, the second probability value may be a probability value corresponding to ST elevation myocardial infarction of the user 1 or a probability value corresponding to non-ST elevation myocardial infarction of the user 1. That is, the processor 130 may determine whether the disease of the user 1 corresponds to either ST elevation myocardial infarction or non-ST elevation myocardial infarction based on the second probability value obtained via the second neural network model 20-2. Meanwhile, since both ST elevation myocardial infarction and non-ST elevation myocardial infarction correspond to types of acute myocardial infarction, information (e.g., second probability value) output via the second neural network model 20-2 may be referred to as information about the first type of the first disease of a first category. That is, the second neural network model 20-2 may be pre-trained to output information about the first type of the first disease of the first category.

For ease of description of the present disclosure, the following description will be given on the assumption that the first type of the first disease is ST elevation myocardial infarction.

Meanwhile, according to one embodiment of the present disclosure, the first disease of the first category may be acute myocardial infarction. Accordingly, when it is determined that the user 1 has myocardial infarction based on the first probability value and it is also determined that the user 1 has ST elevation myocardial infarction based on the second probability value, the processor 130 may generate a diagnosis result for acute myocardial infarction of the user 1.

An embodiment of the present disclosure in which a diagnosis result of the user 1 is generated based on the first and second probability values will be described in detail below.

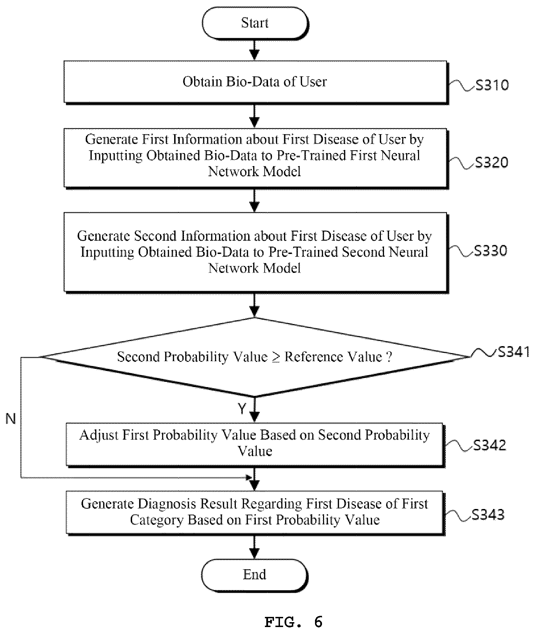

FIG. 6 is a flowchart showing a method for diagnosing a disease of the user 1 by using the first and second neural network models 20-1 and 20-2 according to one embodiment of the present disclosure. Steps S610 to S630 shown in FIG. 6 may correspond to steps S310 to S330 shown in FIG. 3, respectively, so that detailed descriptions thereof will be omitted.

According to one embodiment of the present disclosure, the processor 130 may adjust the first probability value included in the first information based on the second probability value included in the second information, and may generate a diagnosis result for the user 1 regarding the first disease of the first category based on the first probability value.

More specifically, the processor 130 may adjust the first probability value for the presence or absence of the first disease based on the second probability value when the first type of the first disease is identified based on the output value (i.e., the second probability value) of the second neural network model.

As an example, when the first type of the first disease is identified via the second neural network model, it may mean that the probability that the first disease of the first category is present in the user is high. Accordingly, the processor 130 may adjust the first probability value for the presence or absence of the first disease based on the second probability value for the presence or absence of the first type of the first disease.

As an example, when the second probability value is equal to or larger than a reference value, the processor 130 may adjust the first probability value by applying a weight corresponding to the second probability value to the first probability value. In contrast, when the second probability value is smaller than the reference value, the processor 130 may maintain the first probability value.

More specifically, the processor 130 may determine whether the second probability value is equal to or larger than the reference value. In this case, the reference value may be set differently for each user 1 depending on the age, body type, pathological history, and/or the like of the user 1.

As an example, the processor 130 may determine that the second probability value is equal to or larger than the reference value and that the user 1 has the first type of first disease. More specifically, the processor 130 may determine that the second probability value is equal to or larger than the reference value and that ST elevation myocardial infarction is identified based on the electrocardiogram signal of the user 1. Then, when it is determined that ST elevation myocardial infarction is identified, the processor 130 may adjust the first probability value by applying a weight corresponding to the second probability value to the first probability value. In contrast, the processor 130 may determine that the second probability value is smaller than the reference value and that ST elevation myocardial infarction is not identified based on the electrocardiogram signal of the user 1. Furthermore, when it is determined that ST elevation myocardial infarction is not identified, the processor 130 may maintain the first probability value without applying a weight corresponding to the second probability value to the first probability value.

More specifically, when the first probability value is 40, the second probability value is 32, and the reference value is 30, the processor may determine that the second probability value is equal to or larger than the reference value, and may also determine that the user has ST elevation myocardial infarction. In this case, the processor 130 may determine a weight corresponding to the second probability value. For example, when the weight is set to a value 0.5 times the second probability value 32, the processor 130 may obtain an adjusted first probability value (56 = 32 + 16) by applying the weight (16 = 32 × 0.5) to the first probability value (40). In contrast, when the first probability value is 40, the second probability value is 24, and the reference value is 30, the processor may determine that the second probability value is smaller than the reference value, and may also determine that the user does not have ST elevation myocardial infarction. In this case, the processor 130 may maintain the obtained first probability value 40 without change.

Meanwhile, the processor 130 may adjust the first probability value based on the second probability value in various manners. For example, when the second probability value is equal to or larger than the reference value, the first probability value may be changed into the second probability value.

Alternatively, the processor 130 may apply a weight varying depending on the difference between the first probability value and the second probability value. More specifically, when the second probability value is equal to or larger than the reference value and the difference between the first probability value and the second probability value is equal to or larger than a preset difference value, a first weight may be applied. When the second probability value is equal to or larger than the reference value and the difference between the first probability value and the second probability value is smaller than the preset difference value, a second weight that is smaller than the first weight may be applied.

Alternatively, the processor 130 may apply a weight varying depending on one of a plurality of preset sections including the first probability value. More specifically, when the second probability value is equal to or larger than the reference value and the first probability value is included in a first section (equal to or larger than 0 and smaller than a first reference value), a third weight may be applied. When the second probability value is equal to or larger than the reference value and the first probability value is included in a second section (equal to or larger than the first reference value and smaller than a second reference value), a fourth weight that is smaller than the third weight may be applied. When the first probability value is included in the third section (equal to or larger than the second reference value and smaller than a third reference value), a fifth weight that is smaller than the fourth weight may be applied.

Meanwhile, the processor 130 may generate a diagnosis result based on the first probability value. In this case, the first probability value may include the first probability value adjusted based on the second probability value, or may include the first probability value output from the first neural network model (i.e., the maintained first probability value).

As an example, the processor 130 may generate a first diagnosis result corresponding to the first disease of a first category when the first probability value is equal to or larger than a first value, may generate a second diagnosis result corresponding to the first disease of the first category when the first probability value is smaller than the first value and equal to or larger than a second value, and may generate a third diagnosis result corresponding to the first disease of the first category when the first probability value is smaller than the second value.

More specifically, in the case where the first value is set to 3 and the second value is set to 48.5, the processor 130 may generate a diagnosis result indicating that the user is normal (or a diagnosis result indicating that the possibility of acute myocardial infarction is low) when the first probability value (the adjusted first probability value or the maintained first probability value) is smaller than the first value (3). Furthermore, the processor 130 may generate a diagnosis result indicating that the user belongs to a middle risk group for acute myocardial infarction (or a diagnosis result indicating that there is the possibility of acute myocardial infarction) when the first probability value is equal to or larger than the first value (3) and smaller than the second value (48.5). Finally, the processor 130 may generate a diagnosis result indicating that the user belongs to a high risk group for myocardial infarction (or a diagnosis result indicating that the possibility of acute myocardial infarction is high) when the first probability value is equal to or larger than the second value (48.5).

Meanwhile, the processor 130 may compare the first probability value and the second probability value with the first value and the second value, may determine that the user 1 does not have a myocardial infarction disease when it is determined that the first probability value is smaller than the first value, and may determine that the user 1 belongs to a high risk group for myocardial infarction when it is determined that the second probability value is equal to or larger than the reference value. In this case, the processor 130 may identify the electrocardiogram signal of the user 1 as being abnormal, and may generate information requesting the re-measurement of an electrocardiogram signal from the user 1 and transmit the information to the external electronic device 200 via the communication interface 120 or output it via an output interface (e.g., a speaker, a display, or the like).

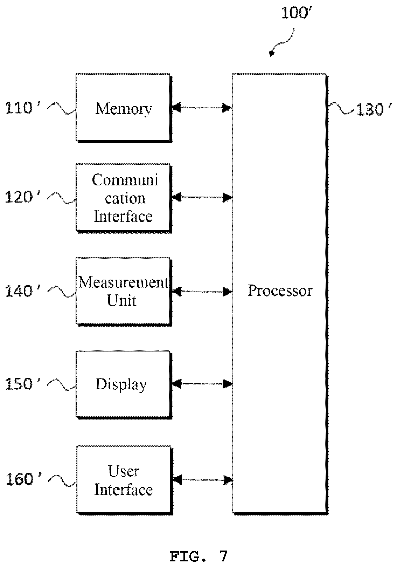

According to one embodiment of the present disclosure, the electronic device 100 may be implemented as an electrocardiogram measurement device. Referring to FIG. 7, the electronic device implemented as an electrocardiogram measurement device according to one embodiment of the present disclosure will be described.

FIG. 7 is a detailed configuration diagram of an electronic device according to an alternative embodiment of the present disclosure.

In this case, an electronic device 100' includes memory 110', a communication interface 120', a measurement unit 140', a display 150', a user interface 160', and a processor 130'. The descriptions of the memory 110, the communication interface 120, and the processor 130 shown in FIG. 2 may be applied to the memory 110', the communication interface 120', and the processor 130' shown in FIG. 7 in the same manner, so that detailed descriptions thereof will be omitted.

The measurement unit 140' includes a plurality of leads configured to be attached to different parts of the body of the user 1, and may generate an electrocardiogram signal of the user 1 based on the voltage difference between the body parts of the user 1 measured via the plurality of leads. For example, the measurement unit 140' may include limb leads and precordial leads. In this case, the limb leads may include four electrodes configured to be attached to the limbs (hereinafter, the limb electrodes). Furthermore, the precordial leads may include six electrodes configured to be attached to the chest (hereinafter, the precordial electrodes).

The limb electrodes may include a right arm electrode (RA), a left arm electrode (LA), a right leg electrode (RL), and a left leg electrode (LL). The right leg electrode (RL) may be a common electrode or a ground electrode. The limb electrodes may be attached to positions corresponding to the right arm, the left arm, the right leg, and the left leg, respectively.

Furthermore, the precordial electrodes may include a first precordial electrode V1, a second precordial electrode V2, a third precordial electrode V3, a fourth precordial electrode V4, a fifth precordial electrode V5, and a sixth precordial electrode V6.

The display 150' may output various types of image information. For example, the processor 130 may output the diagnosis result of the user 1 via the display 150'. Alternatively, the processor 130 may output warning information about the first type of the first disease for the user 1 via the display 150'.

To this end, the display 150' may be implemented as a display including self-luminous elements or as a display including non-luminous elements and a backlight. For example, the display 150' may be implemented as various types of displays such as a liquid crystal display (LCD), an organic light-emitting diode (OLED) display, light-emitting diodes (LEDs), micro LEDs, mini LEDs, a plasma display panel (PDP), a quantum dot (QD) display, and a quantum dot light-emitting diodes (QLEDs).

The display 150' may also include a driving circuit, a backlight unit, etc., which may be implemented in the form of a-si TFTs, low-temperature poly-silicon (LTPS) TFTs, organic TFTs (OTFTs), and/or the like. Meanwhile, the display 150' may be implemented as a touch screen combined with touch sensors, a flexible display, a rollable display, a 3D display, a display in which multiple display modules are physically connected, and/or the like.

In addition, the display 150' may constitute a touch screen together with a touch panel.

The user interface 160' is a component used to perform interaction between the electronic device 100 and the user 1, and the processor 130 may output a diagnosis result via the user interface 160'. Meanwhile, the user interface 160' may include at least one of touch sensors, motion sensors, buttons, a jog dial, switches, and a microphone, but is not limited thereto.

The various embodiments of the present disclosure described above may be combined with one or more additional embodiments and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method performed by an electronic device including at least one processor, the method comprising:
obtaining biometric-data of a user;
generating first information about a first disease of the user by inputting the obtained biometric-data to a pre-trained first neural network model;
generating second information about the first disease of the user by inputting the obtained biometric-data to a pre-trained second neural network model; and
generating a diagnosis result for the user regarding a first category of the first disease based on the first and second information.

2. The method of claim 1, wherein generating the diagnosis result comprises:
adjusting a first probability value included in the first information based on a second probability value included in the second information, and generating a diagnosis result for the user regarding the first disease of the first category based on the first probability value.

3. The method of claim 2, wherein generating the diagnosis result comprises:
when the second probability value is equal to or larger than a reference value, adjusting the first probability value by applying a weight corresponding to the second probability value to the first probability value; and
when the second probability value is smaller than the reference value, maintaining the first probability value.

4. The method of claim 2, wherein generating the diagnosis result comprises:
generating a first diagnosis result corresponding to the first disease of the first category when the first probability value is equal to or larger than a first value, generating a second diagnosis result corresponding to the first disease of the first category when the first probability value is smaller than the first value and equal to or larger than a second value, and generating a third diagnosis result corresponding to the first disease of the first category when the first probability value is smaller than the second value.

5. The method of claim 1, wherein the first information is information about a presence or absence of the first disease, and the second information is information about a first type of the first disease.

6. The method of claim 1, comprising extracting first and second neural network models from among a plurality of neural network models based on the first disease and the category of the diagnosis result.

7. The method of claim 5, wherein:
the biometric-data comprises an electrocardiogram signal;
the first disease comprises any one of myocardial infarction and ischemic heart disease; and
the first type comprises any one of ST elevation myocardial infarction (STEMI) and non-ST elevation myocardial infarction (NSTEMI).

8. The method of claim 1, wherein:
the first and second neural network models are pre-trained based on training data in which a plurality of labels set based on different criteria are assigned for a same electrocardiogram signal; and
the plurality of labels comprises a first-category label corresponding to the presence or absence of the first disease and a second-category label corresponding to the first type of the first disease.

9. The method of claim 1, wherein generating the first information about the first disease and generating the second information about the first disease are performed in parallel.

10. An electronic device, comprising:
a communication interface;
memory configured to store pre-trained first and second neural network models; and
at least one processor configured to obtain biometric-data of a user, generate first information about a first disease of the user by inputting the obtained biometric-data to a pre-trained first neural network model, generate second information about the first disease of the user by inputting the obtained biometric-data to a pre-trained second neural network model, and generate a diagnosis result for the user regarding a first category of the first disease based on the first and second information.

11. A method performed by an electronic device including at least one processor, the method comprising:
obtaining biometric-data of a user;
generating first information about a first disease of the user by inputting the obtained biometric-data to a pre-trained first neural network model;
generating second information about the first disease of the user by inputting the obtained biometric-data to a pre-trained second neural network model; and
generating a diagnosis result for the user regarding a first category of the first disease based on the first and second information.
